# EUROPEAN PATENT SPECIFICATION

(11) **EP 0 402 997 B1**
(45) Date of publication and mention of the grant of the patent: **14.09.1994**
(21) Application number: 90201480.2
(22) Date of filing: 08.06.1990
(51) Int. Cl.: C12Q 1/68, G01N 33/569, G01N 33/576, C12Q 1/70

(54) **Method for determining nucleic acid**
Verfahren zur Bestimmung von Nukleinsäuren
Procédé pour déterminer des acides nucléiques

(30) Priority: 15.06.1989 NL 8901512
(43) Date of publication of application: 19.12.1990
(73) Proprietor: Akzo Nobel N.V., 6824 BM Arnhem (NL)
(72) Inventor: Rijntjes, Petrus Johannes Maria, NL-5282 MG Boxtel (NL); Kuijpers, Leonardus Paulus Clemens, NL-5283 VG Boxrel (NL); Heijtink, Rudolf Arnold, NL-4285 WJ Woudrichem (NL)
(74) Representative: Hermans, Franciscus G.M.

(56) References cited:
- EP-A- 0 118 735
- EP-A- 0 145 356
- EP-A- 0 229 701
- EP-A- 0 235 727
- EP-A- 0 366 448
- BIOLOGICAL ABSTRACTS, vol. 87, no. 1, January 1989, page AB-637, abstract no. 6438, Philadelphia, PA, US; A. BUDKOWSKA et al.: "Occurrence of pre-S1 antigen in viremic and nonviremic carriers of hepatitis B surface antigen", & J. MED. VIROL. 26(2): 217-226, 1988

## Description

The invention relates to a method for the qualitative and/or quantitative determination of nucleic acid in a test fluid and to a test kit for carrying out the determination.

A method for detecting the presence of Salmonella bacteria in foodstuffs is described in USP 4,689,295. For this purpose a test sample is taken from the foodstuff. The Salmonella bacteria is lysed in this sample, by which means bacterial DNA is liberated. Subsequently this bacterial DNA is denatured and is then hybridized under the suitable conditions using a specific DNA probe. By this means it is possible to detect Salmonella DNA. The method which is used for this purpose is fairly laborious. For example, various centrifugation steps as well as various washing steps have to be carried out during the isolation procedure. Moreover, it is necessary to start with a large amount of starting material in order to be able to isolate detectable amounts of bacterial DNA. Furthermore a great disadvantage is that in addition to the bacterial DNA also cellular DNA is isolated from the test sample, as a result of which a very specific probe must be available to enable the bacterial DNA to be detected in the large excess of cellular DNA.

In EP 145356 the preparation of viral DNA from Hepatitis B virus by using a carrier bound monoclonal antibody to a surface epitope of the virus to isolate it from a sample, followed by pronase lysis of bound virus to release its DNA is described.
A method for the detection of a virus by amplification of viral nucleic acid that is extracted and exhaustively dialysed prior to the amplification reaction has been described in EP 229701.

The characteristic feature of the invention is that the test fluid is incubated with a solid carrier provided with an immunochemical component, the carrier binding a biological particle from the test fluid by means of an specific biological interaction such as an immunochemical reaction or a receptor-ligand interaction, which particle contains the nucleic acid to be detected, after which the biological particle bound to the carrier is separated from the test fluid, followed by an incubation of the biological particle bound to the carrier with an agent which breaks open the biological particle and libarates the nucleic acid and that, at the same time or subsequently, the liberated nucleic acid is amplified, after which the presence and/or amount of the nucleic acid is determined, which gives a measure of the presence and/or amount of biological particles in the test fluid.

This method is particularly suitable if the biological particle possesses a protein-containing coat and the solid carrier is provided with a (monoclonal) antibody directed against an epitope of the coat of the biological particle. Biological particles which have a protein coat are, for example, viruses or cells, possibly cells infected with one or more viruses, such as T-cells. The nucleic acid in the biological particle can belong to the genetic composition of the particle itself. The nucleic acid can also belong to the genetic composition because the nucleic acid has penetrated or has been introduced into that particle or has been entrapped by that particle. Single-cell organisms can also be detected by means of the method according to the invention.

The method is specifically suitable for infectious viruses, such as Hepatitis virus and viruses which cause AIDS (for example HIV-1, HIV-2). In combination with a suitable (monoclonal) antibody directed against an epitope of the virus, the method gives a markedly good result.

If one uses a monoclonal antibody anti-preSl, it is possible, specifically, to detect infectious Hepatitis B virus particles (the so-called Dane particles) by means of the method according to the invention. Infectious Dane particles (42 nm) contain a relatively large amount of preS1 and preS2 in contrast to so-called "dummy" particles of 22 nm, which contain hardly any preS1 and preS2 but do contain a large amount of S-protein and no nucleic acid (Summers, J. Replication of hepatitis B viruses. In Viral Hepatitis and Liver Disease, edited by Vyas G.M., Dienstag J.L., Hoofnagle, J.H., New York 1984).

The invention also relates to a test kit for carrying out the method according to the invention. The test kit contains a solid carrier - provided with an immunochemical component - as well as an agent for splitting biological particles and reactants for amplifying nucleic acid.

"A solid carrier provided with an immunochemical component" is understood to mean a solid carrier to which the immunochemical component is bound directly or indirectly.

Since the introduction of the polymerase chain reaction (PCR) with Klenow DNA polymerase (Saiki et al., 1985, Science, 230 p. 1350-1354) and recently with Taq polymerase (Saiki et al., 1988, Science, 239, p. 487-491), many research groups have used this method to detect viral sequences or have used this method in molecular cloning techniques. This amplification method which can replicate DNA sequences by a factor of 10⁵ to 10⁷ is of great importance if only a test fluid is available which contains amounts of nucleic acid which are too small to be detectable without amplification.

If one uses the method described in the previously mentioned USP 4,689,295 in order to isolate nucleic acid from a test sample in such a manner, which is currently widely used, a nucleic acid amplification system, such as PCR, used thereafter has disadvantages. Specifically, as a result of a-specific hybridization with the primers to be used in an amplification system of this type, undesired sequences will also be co-replicated. It is known that in the PCR technique the end product is its own specific template. This means that every trace of contamination with primer-hybridizing material will lead to an erroneous positive signal. In the light of this it is clear that very special precautionary measures must be employed if an amplification technique of this type is to be usable in practice. Some of these measures are, for example, that pipettes must be reserved for this special use, glassware must be cleaned exceptionally carefully and the molecular biological recombinant work, inherently to the particles to be detected, should preferably be carried out in a laboratory area which is isolated from the area in which the amplification technique takes place. Despite many precautionary measures, negative test fluids are nevertheless still frequently found to give a positive signal.

A great advantage of the method according to the invention is now that the entire procedure can be carried out in one (small) reaction vessel, for example in a well of a microtitration plate. Moreover, only a minimum of reaction steps have to be carried out to perform the method. As a consequence the risk of contamination is reduced. Since the test fluid is removed after the biological particles containing the nucleic acid to be detected have been bound to the solid carrier, the nucleic acid which has passed into the test fluid as a result of contamination is also removed. The invention is explained below with the aid of a detailed description.

It is presumed that the virus responsible for the disease is present in a test fluid, for example urine or blood or blood products.

This virus consisting of at least a protein coat and a nucleic acid, DNA or RNA, is bound to a solid carrier by means of an antibody which is specifically directed against an epitope of the virus. By this means the virus is specifically separated from the other components present in the test fluid. One such component is, for example, cellular material.

The binding of a virus of this type to a solid carrier can take place by bringing an antibody, which has already been coupled to the solid carrier beforehand, into contact with the test fluid.

An alternative for binding virus (antigen) to a solid carrier is a method in which the biological particles, for example virus particles, are brought into contact with antibodies which are not bound to a carrier, which antibodies during or after the immunochemical reaction are bound to a solid carrier having affinity for these antibodies.

Also a possibility to bind virus (antigen) to a solid carrier is a method in which the virus particles, containing like for instance in HIV the gp160 as epitope/ligand, are captured by a CD4 receptor which receptor is already bound to the solid phase.

The solid carrier to which an immunochemical component is bound directly or indirectly can be any material of any shape and size which makes it possible to separate (fluid) phase bound to the carrier from another phase not bound to the carrier. The direct or indirect binding of the antibody or antibodies to a solid carrier can take place covalently or ionically or with the aid of hydrogen bridges or can be based on, for example, van der Waals interaction or immunochemical reaction. Suitable carriers are, for example, test tubes, filter paper, wells of microtitre plates or strips, or small rods, spheres or discs made from, for example, glass, silica or plastic.

The separation between the phase bound to the carrier, to which phase the biological particles are bound, and the fluid phase can take place in the conventional manner. For example by drawing off or decanting the fluid is removed, after which one or more washing steps can be carried out if necessary.

The biological particles bound by antibody-antigen complexes to a solid carrier are then incubated with an agent which breaks open the biological particles and liberates the nucleic acid present in the biological particles. Agents which can be used for breaking open a coat on the biological particles, frequently a protein, are protein-splitting enzymes such as pronase or proteinase K. Further suitable agents are, for example, detergents such as sodium dodecylsulphate, sodium dodecyl-N-sarcosinate, cetyltrimethylammonium bromide, dodecylpyrimidinium chloride, palmitoyllysolecithin, dodecyl-N-betaine, polyoxyethylene alcohols, polyoxyethylene isoalcohols, polyoxyethylene p-t-octylphenols, polyoxyethylene nonylphenols, polyoxyethylene esters of fatty acids, polyoxyethylene sorbitol esters, sodium dodecylsulphonate, tetra-decylammonium bromide and saponins. The non-ionic detergents are preferred. Very suitable non-ionic detergents are the polyoxyethylene derivatives, including Bry^{(R)}, Triton^{(R)}, Nonidet P40^{(R)} and Tween^{(R)}.

Another method for breaking open a coat on the biological particles is heat treatment and/or alkaline treatment.

After the breaking-open of the protein coat, the nucleic acid, DNA or RNA, is liberated into the reaction mixture. This reaction mixture is then treated, simultaneously or successively, with reactants to amplify the liberated nucleic acid. Any suitable nucleic acid amplification technique can be used for this purpose, such as the previously mentioned PCR technique (described in USP 4,683,202), the so-called TAS technique (described in WO 88/10315) or the so-called NASBA technique (described in EP 329.822).

After carrying out a suitable amplification technique, the presence and/or amount of the nucleic acid is determined in accordance with a manner which is in itself known, for example via a direct or indirect spectrophotometric determination, preferably in the same (small) reaction vessel as that in which the nucleic acid was liberated. A conventional gel electrophoresis method followed by a Southern blotting can also be used. The amplified nucleic acid can also be detected by making use of labelled primers during an amplification reaction. By this means the determination is a measure for the presence and/or amount of biological particles in the test fluid. The invention is explained in more detail with the aid of the example which follows hereinafter.

### Example I

### a. Binding of Hepatitis B virus to anti-preS1 on a solid phase

50 µl of serum (negative or containing 10 pg Hepatitis B virus (HBV) as positive control) are incubated for 1 hour at 37^{o}C in a well of a microtitration plate. This well has been coated with anti-preS1 directed against the preS1 epitope on HBsAg. After washing 5 times with PBS-Tween^{(R)}, the inside of the well is treated with alkali, either direct or after a Proteinase K treatment. Proteinase K treatment is carried out by adding 50 µl of 0. 01% (W/V) Proteinase K (Merck, 27IU/mg) in 10 mmol/l Tris/HCl pH 7.5, 1 mmol/l EDTA, 0.1% SDS and the mixture is incubated at 37^{o}C for 1 hour. Alkali treatment is carried out by adding 2.5 µl of NaOH (final concentration 50 mM or 500 mM) and then incubating for 15 minutes at 50^{o}C, after which the solution is neutralized by adding 2.5 µl of HCl of suitable molarity.

### b. Amplification (PCR technique).

50 µl addition of a mixture containing: 20 mmol/l Tris.HCl, pH 8.3, 12 mmol/l MgCl₂, 0.02% (W/V) gelatin, 400 µmol/l each of dATP, dCTP, dGTP and TTP, 2 µmol/l of each primer and 4 units of Taq polymerase are added and the mixture is incubated for 1 minute at 94^{o}C followed by 4 minutes at 65^{o}C. This cycle of incubating at 94^{o}C and then at 65^{o}C is repeated 40 times. The primers used are the sequences indicated below. The complementary sequence is localized around the unique EcoRI restriction endonuclease cutting site in the HBV genome.
These sequences are 5'CCTCCTGCCTCCACCAATCG-OH and 5'GAACTGGAGCCACCAGCAGG-OH.

### c. Agarose gel electrophoresis/hybridization

After the abovementioned PCR technique, 3 µl of the solution are applied to a 2% agarose gel, after which a gel electrophoresis takes place for 2.5 hours with 50 mA. The gel is then coloured with ethidium bromide. After Southern blotting, the nitrocellulose filters are hybridized with an internal probe labelled with ³²P and washed under stringent conditions, followed by auto-radiography. It is found from this detection method that the method according to the invention leads to a highly sensitive (femtogram level) technique which can be carried out in a single small reaction vessel.

## Claims

1. Method for the specific detection of infectious Hepatitis B virus in a test fluid, where the test fluid is incubated with a solid carrier provided with anti-preS1 antibodies, after which the solid carrier, to which virus particles have bound, is seperated from the test fluid and viral nucleic acid is liberated from the particles and amplified, after which the presence and/or amount of nucleic acid is determined.

2. Test kit for carrying out the method according to claim 1, containing a solid carrier provided with anti-preS1 antibodies, an agent for splitting biological particles and reactants for amplifying nucleic acid.

## Patentansprüche

1. Verfahren zum spezifischen Nachweis von infektiösen Hepatitis B-Viren in einer Testflüssigkeit, wobei die Testflüssigkeit mit einem festen Träger inkubiert wird, der mit anti-preS1-Antikörpern versehen ist, wonach der feste Träger, an den die Viruspartikel gebunden haben, von der Testflüssigkeit getrennt wird und virale Nukleinsäuren aus den Partikeln freigesetzt und vermehrt werden, wonach die Anwesenheit und/oder die Menge der Nukleinsäuren bestimmt wird.

2. Testgarnitur zur Ausführung des Verfahrens gemäss Anspruch 1, die einen festen, mit anti-preS1-Antikörpern versehenen Träger, ein Agens zum Spalten biologischer Partikel und Reagenzien zum Vermehren von Nukleinsäuren umfasst.

## Revendications

1. Procédé permettant la détection spécifique du virus de l'hépatite B infectieux dans un liquide à tester, dans lequel le liquide à tester est incubé avec un support solide pourvu d'anticorps anti-preS1, puis le support solide auquel les particules virales sont liées est séparé du liquide à tester et l'acide nucléique viral est libéré des particules et amplifié après quoi la présence et/ou la quantité de l'acide nucléique est déterminée.

2. Kit de test permettant de mettre en oeuvre le procédé selon la revendication 1, contenant un support solide pourvu d'anticorps anti-preS1, d'un agent permettant la rupture des particules biologiques et de réactifs permettant l'amplification de l'acide nucléique.
